# EUROPEAN PATENT SPECIFICATION

(11) **EP 2 255 834 B1**
(45) Date of publication and mention of the grant of the patent: **09.05.2012**
(21) Application number: 10165816.9
(22) Date of filing: 08.11.2005
(51) Int. Cl.: A61L 27/36, A61L 27/38, A61L 27/50

(54) **Scaffold engineering with homing factors**
Gewebeaufbaugerüste mit Homingfaktoren
Échafaudage pour ingénierie tissulaire avec des facteurs d'homing

(30) Priority: 08.11.2004 GB 0424560; 11.03.2005 US 660766 P
(43) Date of publication of application: 01.12.2010
(62) Divisional of application: 05808071.4
(73) Proprietor: K.U. Leuven Research and Development, 3000 Leuven (BE); AlphaGen N.V., 3600 Genk (BE)
(72) Inventor: Flameng, Willem, 3500, Oud-Heverlee (BE); De Visscher, Geofrey, 2800, Antwerpen (BE)
(74) Representative: Bird, Ariane

(56) References cited:
- WO-A-2004/090120
- US-A- 6 082 364
- US-A1- 2001 051 824
- DAI ET AL: "Stem cell transplantation for the treatment of myocardial infarction" TRANSPLANT IMMUNOLOGY, ELSEVIER, vol. 15, no. 2, December 2005 (2005-12), pages 91-97, XP005246068 ISSN: 0966-3274
- HENG B C ET AL: "Scaffold implants for the controlled release of heparan sulfate (HS) and other glycosaminoglycan (GAG) species: This could facilitate the homing of adult stem cells for tissue/organ regeneration" MEDICAL HYPOTHESES, EDEN PRESS, PENRITH, US, vol. 65, no. 2, 2005, pages 414-415, XP004909564 ISSN: 0306-9877
- HENG B C ET AL: "Combining transfusion of stem/progenitor cells into the peripheral circulation with localized transplantation in situ at the site of tissue/organ damage: A possible strategy to optimize the efficacy of stem cell transplantation therapy" MEDICAL HYPOTHESES, EDEN PRESS, PENRITH, US, vol. 65, no. 3, 2005, pages 494-497, XP004969659 ISSN: 0306-9877

## Description

### Field of the Invention

The present invention relates to tissue engineering and to cell seeding of scaffolds of implantable devices. The invention further relates to molecules which ensure in vitro and/or in vivo seeding of matrix with cells.

### Background

Prosthetic heart valves suffer from possible complications such as thrombosis, endocarditis, mechanical failure, tissue degradation, calcification. These problems and the fact that these prostheses lack growth and remodeling potential in pediatric patients are the motivation for tissue engineering of heart valves.

Tissue engineering's primary goal is the restoration of function by delivery of living elements which become incorporated into the patient. Tissue engineering is essentially based on 3 elements:
- cells, representing the living component
- matrix, providing a three dimensional support structure
- signal molecules which influence gene expression and extracellular matrix deposition.

Tissue engineering combines cells and scaffolds to construct new tissue. For cell seeding of matrices, two options are currently being investigated: cultured autologous cells for *in vitro* seeding and the attraction of autologous cells *in vivo.*

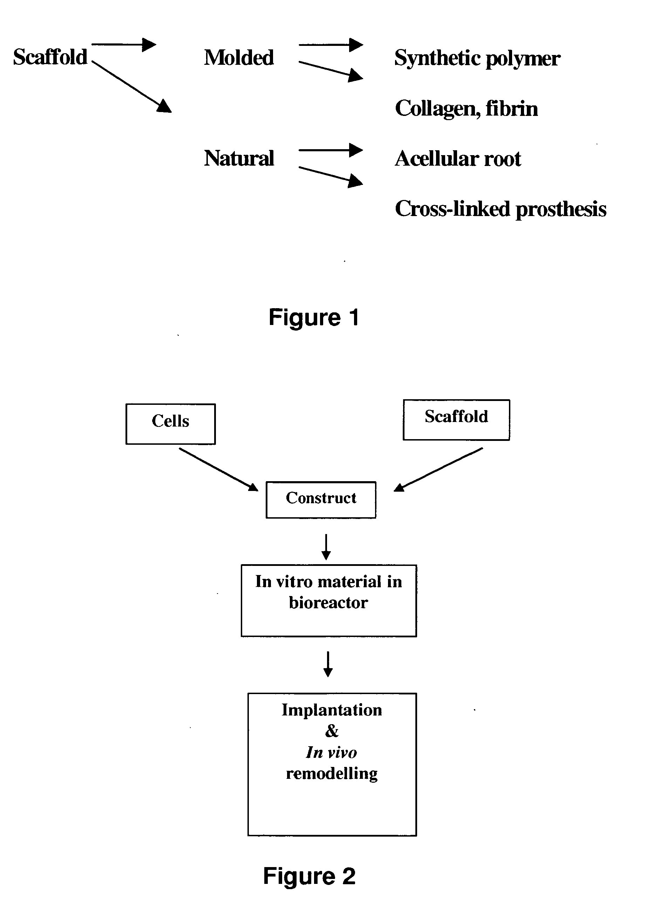

Different matrices are being considered for tissue engineering (see Figure 1). A matrix can be a molded scaffold of a synthetic polymer or of collagen or fibrin. The scaffold can be natural (eg acellular root) or can be a cross-linked prosthesis.

In addition, different processes have been devised in order to obtain a viable tissue engineered heart valve. The complete paradigm describes the construction of a valve by combining cells and scaffold *in vitro,* followed by *in vitro* maturation of the construct in a bioreactor (Figure 2). The mature construct can then be implanted in the patient and possibly undergo in vivo remodeling (reviewed in Rabkin & Schoen (2002) Cardiovasc. Pathol. 11, 305-317). The most thoroughly studied construct is a valve created by Hoerstup et al. ((2002) Circulation 106, I143-I150)). They succeeded in obtaining a viable and remodeled sheep pulmonary valve using the complete paradigm with a bioreactor.

Campbell al. ((1999) Circ. Res. 85, 1173-1178) have suggested ensuring seeding of scaffolds in tissue engineering of blood vessels making use of a known defense-mechanism, namely the foreign body reaction. A mature foreign body reaction is comprised of a macrophage layer, several layers of fibroblasts and an external mesothelial layer (Butler et al. (2001 a) Biomed. Sci. Instrum. 37, 19-24.; Butler et al. (2001 b) J. Invest Surg. 14, 139-152). Although Campbell et al. (1999, cited above) assert the fibroblast are derived from trans-differentiated macrophages, no conclusive evidence has been published yet.

In order to be successful, these methods have to meet a number of regulatory challenges, including optimal function and durability. In order to justify the high cost of tissue-engineered valves, they must be demonstrated to have superior qualities over existing valves. Additionally all of the above-mentioned techniques have to overcome the challenge of bio-safety. The biosafety issues and guidelines in that respect of the FDA are well-documented ("Guidance on applications for products comprised of living autologous cells manipulated ex vivo and intended for structural repair or reconstruction". 95N-0200 (1996); "Proposed approach to regulation of cellular and tissue-based products - The food and drug administration". Journal of Hematotherapy 6: 195-212 (1997); "Guidance for industry - Guidance for human somatic cell therapy and gene therapy." Human Gene Therapy 9: 1513-1524 (1998); "PHS guideline on infectious disease issues in xenotransplantation". (2001); "Transmissible spongiform encephalopathies advisory committee meeting." (2001)).

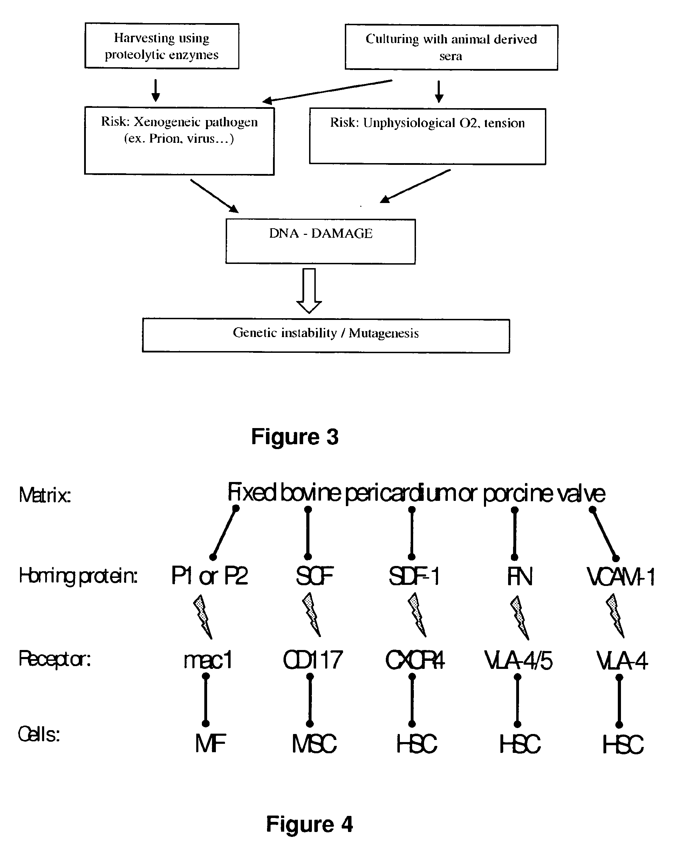

The use of *in vitro* cultured or even harvested cells posess specific problems. As illustrated in Figure 3, both harvesting and culturing of cells can induce genetic instability or mutagenesis. This can be attributed to different factors. Both harvesting and culturing generally require the use of foreign proteins. The xenogeneic origin of proteolytic enzymes or sera, are a potential source of interspecies pathogen contamination. Such contamination, if undetected, not only puts the culture itself at risk but also the recipient. The proliferation mechanism of certain viruses can allow the insertion of their genome into the host genome, which can cause deleterious mutations depending on the site of insertion. Certain viral genes can also act as oncogenes. Bio-safety issues equally apply to the use of xenogeneic materials. Besides the risk of viral infections, cultivation of cells also exposes cells to unphysiological conditions such as increased oxygen tension. On average mammalian tissue is exposed to an oxygen tension ranging from 2-8 % whereas generally incubators use compressed air with a 21 % oxygen tension. It has been demonstrated that primary murine fibroblasts are extremely vulnerable to DNA damage resulting in senescence and spontaneous immortalisation. Despite the fact that in these experiments human fibroblasts were much less affected, other studies demonstrate that human cells are not insensitive to oxidative stress. For example, human articular cartilage chondrocytes have been demonstrated to be sensitive to oxidative stress. Using cell lines, it has been demonstrated that oxygen predominantly induces genome rearrangements in rapidly proliferating cells. Moreover it has been demonstrated that human fibroblasts also senescence in response to oxidative stress, which can cause telomere shortening and single strand breaks in the telomeric DNA at a rate depending on the oxidative stress. In fibroblast cultures, an increase in population doublings was obtained when the oxygen tension was decreased. Another remarkable observation is that senescence upregulates eight genes among which fibronectin, osteonectin and alpha1-procollagen.

Bio-safety is a very important aspect of the regulatory challenges for *in vitro* cell-seeded heart valves and imposes the development of a stringent quality control for each individual valve prosthesis before implantation into the recipient. The increased regulatory challenges will also markedly increase the costs of such prosthesis, thereby limiting its use to specific groups of patients.

There is a need in the art for a way to obtain viable tissue engineered heart valves and other implantable devices. More particularly there is a need for scaffolds which are suitable for cell seeding, particularly cell seeding in vivo, most particularly where cell seeding needs to occur under conditions of increased shear stress, such as scaffolds of heart valves and blood vessels.

### SUMMARY OF THE INVENTION

The present invention relates generally to the use of homing factors in the generation of scaffolds of tissues and organs used for implantation into the animal or human body. According to a particular aspect, the present invention relates to the use of homing factors in the generation of scaffolds susceptible to high shear stress upon implantation into the body. Most particularly the invention relates to scaffolds for use in the cardiovascular system, lymphatic system or other vessels such as urethra. A specific embodiment of the present invention relates to scaffolds comprising a structural matrix coated with one or more homing factors. More particularly the homing factors of the present invention are factors capable of binding to stem cells or progenitor cells.

A particular aspect of the present invention relates to scaffolds of implantable devices intended for prolonged use and function within the body, i.e. based on a durable biocompatible but non-biodegradable matrix. According to a specific embodiment, the structural matrix is a non-crosslinked prosthesis or an acellularised aortic roots.

A further particular aspect of the present invention relates to scaffolds comprising one or more homing factors wherein the homing factor is a ligand of receptor, or a fragment thereof comprising the receptor binding domain, or a peptide which binds to the receptor. Most particularly, the receptor is a receptor expressed on stem cells or progenitor cells.

Particular embodiments of the homing factors or homing proteins used in the context of the present invention are stromal derived factor 1, stem cell factor, VCAM-1, P1 region of fibrinogen and P2 region of fibrinogen, more particularly stromal derived factor 1 (SDF-1) or stem cell factor (SCF) or fragments or derivatives thereof.

In a particular embodiment the scaffold matrix is coated with one or more homing factors and a protein facilitating the interaction between a cell and the matrix of the scaffold. Such protein can be, but is not limited to fibronectin, collagen or fibrinogen. In a particular embodiment wherein the homing protein is VCAM-1 the interaction between VCAM-1 and its receptor on a cell is optionally further enhanced by addition of the pleiotropic protease inhibitor alpha2-macroglobulin.

In a particular embodiment of the invention the homing factor(s) is(are) chemically cross-linked to the structural matrix by way of a linker arm. Herein a linker arm is interspaced between the homing factor and the structural matrix. The binding between homing factor and matrix can be irreversible (permanent) or biodegradable.

According to a further embodiment of the present invention, the homing factor(s) of present invention can be in the form of a fusion protein with a protein facilitating the binding to the structural matrix (i.e. two proteins form one consecutive polypeptide chain).

In a particular embodiment the structural matrix of the scaffold of the present invention is a biological material such as cross-linked bovine pericardium or porcine aortic roots.

In yet a further embodiment the homing factor is chemically cross-linked to the matrix of the scaffold of the present invention.

The present invention also relates to methods for preparing the scaffold of the present invention whereby the matrix of the scaffold is coated with one or more homing factors. According to a particular embodiment, the coating is ensured by impregnation, i.e. by incubation of the matrix in a solution comprising one or more of the aforementioned homing factors in an appropriate impregnation buffer (for example: phosphate buffered saline). Particular embodiments of this method include a pre-coating step with one or more proteins facilitating the interaction between the homing factor and the structural matrix.

According to a particular embodiment, the scaffolds of the present invention do not comprise chemo-attractant factors or mobilisation factors. Alternatively, the scaffolds of the present invention comprise one or more homing factors of the present invention in combination with one or more chemo-attractant factors and/or mobilization factors.

Accordingly, the present invention provides kits comprising a structural matrix, optionally in the form of an implantable device, such as but not limited to a blood vessel or a cardiac valve, and one or more homing factors.

The invention accordingly relates to the use of homing factors to enhance the *in vivo* seeding on a scaffold suitable for replacing a disordered or diseased tissue or organ. The use of homing factors has particular advantages in the generation of scaffolds for implantation at a location which is under high shear stress, e.g. a scaffold for use in the cardiovascular system such as a heart valve or a vascular graft.

### DETAILED DESCRIPTION OF THE INVENTION

The present invention discloses scaffolds, more particularly scaffolds for implantable devices comprising a matrix which, by the presence of one or more homing factors will ensure the binding of appropriate cells thereto. A "scaffold" as used herein relates to an implantable medical device for tissue repair, restoration, augmentation, or regeneration or to replace a diseased, damaged, missing, or otherwise compromised, tissue or organ in the body of a patient. An "implantable medical device" as used herein refers to any device which is intended to be introduced and optionally implanted into the human body, including devices used for implantation into vessels, ducts or body organs, such as a stent, catheter, canunula, vascular or arterial graft sheath, a device for implantation into the oesophagus, trachea, colon, biliary tract, urinary tract, orthopaedic devices, etc.

Of particular interest within the context of the present invention are scaffolds which are intended for locations whereby shear stress will work against natural adherence of appropriate cells to a matrix. Thus of particular interest in the context of the present invention are scaffolds for use in the replacement and/or restoration of tissues which is susceptible to high shear stress such as a blood vessel or a heart valve, the ureter, the biliary ducts, the pancreatic ducts, the cystic, hepatic, or common bile ducts, and the like.

Seeding of such scaffolds can be ensured *in vivo*, *in situ* (i.e. upon implantation at the site of the diseased or damaged artery) *in vivo*, *ex situ* (i.e. implantation in another site in the body) or *in vitro* (e.g. in a bioreactor).

Homing proteins or homing factors are defined as docking molecules which interact with one or more specific cell types and thus, when attached to a matrix, allow the enrichment of those cells types on the matrix. Such a docking molecule ensures the interaction between the matrix and a molecule at the surface of the cell, such as another protein, or a carbohydrate structure, also referred to herein as the cellular target molecule. According to a particular embodiment of the invention, the homing factor is a molecule which ensures a specific interaction between the matrix and one or more specific cell types; this can be ensured by the binding of the homing factor to a molecule which occurs essentially only on the surface of one or more specific cell types or by adjusting the homing factor so as to bind only to the cellular target molecule on the one or more specific cell types.

Typically the interaction between the homing factor and the cell is a ligand-receptor interaction. Thus, a particular embodiment tof the invention relates to scaffolds comprising one or more ligands of receptors which bind to a certain cell type, more particularly the cell types described hereinbelow.

According to another embodiment, homing factors are molecules which specifically bind to carbohydrate structures which are most particularly specifically expressed on stem cells or progenitors cells. Portions of these carbodyrate binding proteins with retain their sugar-binding properties are equally suitable to functions as homing factor.

A homing factor suitable for the scaffolds and methods of the present invention can be a truncated protein and/or a derivative such as a mutated protein as long as it it retains its ability to bind to the cellular target molecule. Minimal binding regions of a homing protein can be defined by mapping truncating deletions. According to a particular embodiment of the present invention, the cellular target molecule is a receptor and the homing factor is a fragment of a ligand thereof, such as a receptor-binding fragment thereof. Typically, homing factors which are derivatives or mutated forms of protein ligands or fragments thereof have at least 80%, particularly at least 90%, most particularly at least 95% amino acid sequence identity to the natural ligand or fragment thereof while retaining the ability to bind to the cellular target molecule.

The homing factors of the present invention ensure and/or increase the adherence of cells to a matrix under varying conditions. The binding of cells through homing factors of the present invention provide particular advantages for seeding of scaffolds of tissues in conditions of high shear stress.

The cells which are captured by way of the homing factors of the present invention are cells which are of interest in the generation of an appropriate scaffold, i.e. a scaffold which can function similarly to the tissue or organ it replaces. According to a particular embodiment of the present invention the homing factor is a protein capable of binding a stem cell or a progenitor cell (i.e. non-differentiated but committed to one or more cell lineages), more particularly haematopoietic progenitor cells.

According to a particular embodiment of the present invention the homing factor is a molecule capable of binding a mesenchymal or hematopoietic stem cell. The present invention demonstrates that the homing of stem cells and/or progenitor cells on a matrix will ensure a seeded matrix scaffold with cells which differentiate into *inter alia* myofibroblast cells.

Particularly suitable homing molecules are of the group consisting of the P1 and P2 epitopes of fibrinogen, stem cell factor (SCF), stromal derived factor 1 (SDF-1), fibronectin (FN) and vascular cellular adhesion molecule-1 (VCAM-1) (figure 4), most particularly SDF-1 or SCF or fragments or derivatives thereof retaining its receptor binding affinity.

A particular embodiment of the present invention relates to the use of SDF-1 is as a homing protein for seeding of a matrix. SDF-1 (stromal cell dervied factor 1) is also known as Pre-B cell growth-Stimulating Factor (PBSF) or as chemokine, cxc motif, ligand 12 (CXCL12). The sequence of SDF-1 cDNA and protein are respectively present in Genbank under Accession Numbers E09668 and NP_001029058. SDF-1 exists in two different forms of 68 amino acids (alpha) and 72 amino acids (beta) respectively, wherein the beta form has 4 additional aminoacids at the carboxyterminus compared to the alpha form. SDF-1 is the ligand of the CXCR4 receptor (Bleuel et al. (1996) Nature 382, 829-833). The importance of an intact N-terminus of SDF1 for receptor binding is documented [e.g. Sadir J Biol Chem. 2004 279, 43854-43860]. Thus for the present invention, fragments of SDF-1 are fragments which retain the aminoterminus of the protein. According to a particular embodiment, a fragment or derivative of SDF-1 is on which contains the aminoterminal region (amino acids 1-14) and the central beta sheet (amino acids 15-54) but which lacks one or more amino acids from the carboxyterminal region (amino acids 55-68 and 55-72 of the alpha and beta from, respectively). The receptor binding activity of such fragments can be evaluated as describe in Sadir et al. (cited above).

According to yet another embodiment of the present invention, SCF is used as a homing protein in a matrix. SCF is recognized by bone marrow mesenchymal stem cells (MSC) via their protein tyrosine kinase receptor (c-kit) in mouse or CD117 in humans (Jiang et al. (2002) Nature 418, 41-49; Nakamura et al. (2004) Exp. Hematol. 32, 390-396) and can thus ensure homing of MSC. Additionally CD117 is an essential factor in the development of haematopoietic progenitor cells (Agis et al. (1993) J Immunol. 151, 4221-4227). SCF (Stem Cell Factor) is also known as KIT Ligand, (KITLG) mast cell growth factor (MGF), and Homolog Of Steel Factor (SF). The cDNA and protein sequence of SCF are deposited in Genbank under Accession number M59964. SCF is the ligand for the KIT tyrosine kinase receptor. SCF exists naturally as an membrane-anchored or as soluble isoforms as a result of alternative RNA splicing and proteolytic processing. According to a particular embodiment of the invention a fragment or derivative of SCF comprises the aminoterminal fragment of 189 amino which contains the extracellular domain of SCF. Alternatively, a fragment or derivative of SCF comprises the naturally occurring soluble form which contains the aminoterminal 165 amino acids of SCF. According to yet another particular embodiment, the fragment or derivative of SCF comprises the aminoterminal 141 residues which contain the receptor binding core of SCF. The numbering of these fragments refers to protein sequence of 248 amino acids which is released after cleavage of the leader sequence. The above-mentioned fragments of SCF and their receptor binding capacity are described in Langley et al. (1994) Arch Biochem Biophys. 311, 55-61. SCF or SCF fragments or derivatives of the present invention can be monomeric or dimeric. Dimeric fragments can be obtained by oxidising or crosslinking cysteine residues which are involved in dimer binding. Alternatively, SCF or its fragments are recombinantly expressed in tandem with a spacer peptide in between them.

A further particular embodiment of the present invention relates to the use of VCAM-1 as homing protein. The cDNA and protein sequence of VCAM-1 are deposited in Genbank under Accession number M60335.

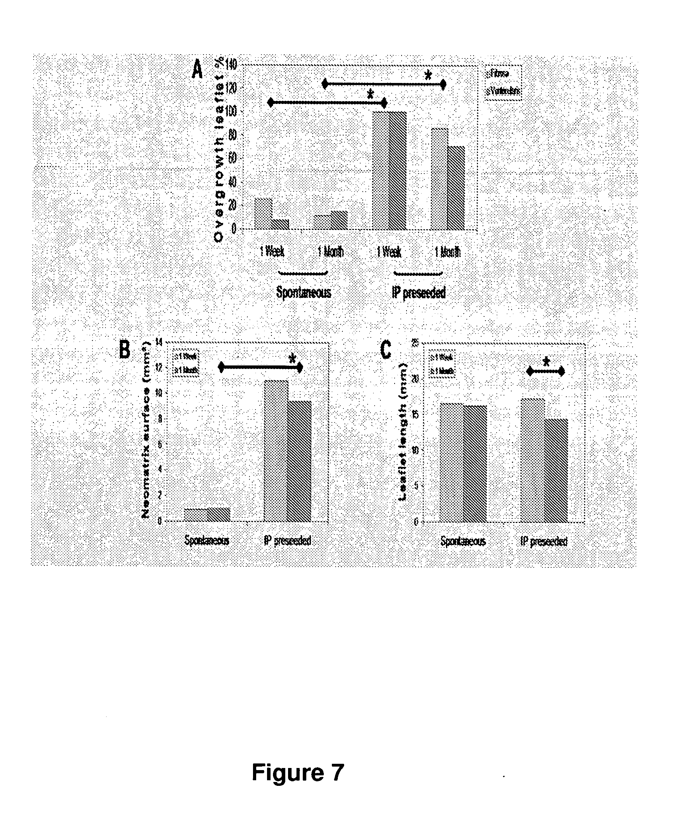

A further particular embodiment of the invention relates to the combined use of a homing factor and a molecule which influences the interaction between the homing molecule and its cellular target molecule (also referred to herein as facilitating protein, see below). According to one embodiment VCAM-1 is added to the matrix in combination with alpha2-macroglobulin (Figure 7), because this pleiotropic protease inhibitor can stabilize the binding between VCAM-1 to VLA4. This function is a result of the inhibition of pleiotropic protease which degrades the VCAM-1 protein (Levesque et al. (2001) Blood 98, 1289-1297). The cDNA and protein sequence of alpha2-macroglobulin are deposited in Genbank under Accession number NM_000014.

According to another particular embodiment, the P1 and P2 epitopes of fibrinogen are used as homing proteins. P1 and P2 are bound by the mac1-integrin of macrophages (MF). It was shown that the foreign body reaction is initiated by adsorption of fibrinogen to the foreign surface. This induces conformational changes of the molecule resulting in exposure of 2 epitopes P1 and P2 (Hu et al. (2001) Blood 98, 1231-1238). The bound macrophages will then attract stem cells as they do in a "standard" foreign body reaction. One embodiment of present invention is a suitable matrix comprising P1 and P2 epitopes to attract endogenous stem cells and suitable for direct replacement of a deficient, diseased or disordered heart valve. The P1 and P2 epitopes can be linked to the matrix. The invention also involves the use of P1 and P2 epitopes to coat a scaffold with said P1 and P2 epitopes to attract endogenous stem cells after implantation. As described by Hu and et al. (2001, cited above), the P1 epitope refers to amino acids 190 to 202 of fibrinogen gamma, while. the P2 epitope refers to amino acids 377 to 395 of fibrinogen gamma.

According to yet another embodiment of the present invention, the matrix comprises fibronectin to home VLA 4 or VLA-5 expressing progenitor cells. The different splice variants of human fibronectin (FN1) are listed in the NIH nucleotide database under accession nr.: NM_212482 (variant 1), NM_212475 (variant 2), NM_002026 (variant 3), NM_212478 (variant 4), NM_212476 (variant 5), NM_212474 (variant 6) and NM_054034 (variant 7).

Particularly suitable for use in the scaffold of the present invention is a matrix or scaffold comprising fibronectin and/or VCAM-1 and further comprising SDF-1 to synergistically act with fibronectin and/or VCAM-1.

The use of homing proteins stromal derived factor 1, stem cell factor, VCAM-1, P1 region of fibrinogen or P2 region of fibrinogen, characterized above, and of any functional homologue or derivatives thereof currently in the art or available to the man skilled in the art, as homing agents in scaffolds for heart valves or blood vessels, is part of this invention.

In theory, the homing factors of the present invention can be obtained from the recipient of the scaffold, but for practical purposes it will be more likely that the homing factors are obtained synthetically or recombinantly (from either pro- or eukaryotic organism). All of the aforementioned homing proteins are commercially available. Recombinant human SDF-1 and recombinant human SCF (E. coli) can be obtained from differentcompanies (Sigma RBI, R&D systems, Campro and Calbiochem). Recombinant human VCAM-1 (mouse myeloma cell line) is available from R&D systems. Native fibronectin derived from human fibroblasts is available from Sigma RBI and Calbiochem. Native fibrinogen derived from human plasma is available from Sigma RBI and Calbiochem.

Regarding the P1 and P2 epitopes of fibrinogen, the epitopes can be derived from the native fibrinogen by either proteolytic cleaving of the native protein, but more preferably by in vitro biosynthesis following the protein sequence described by Hu et al. (cited above).

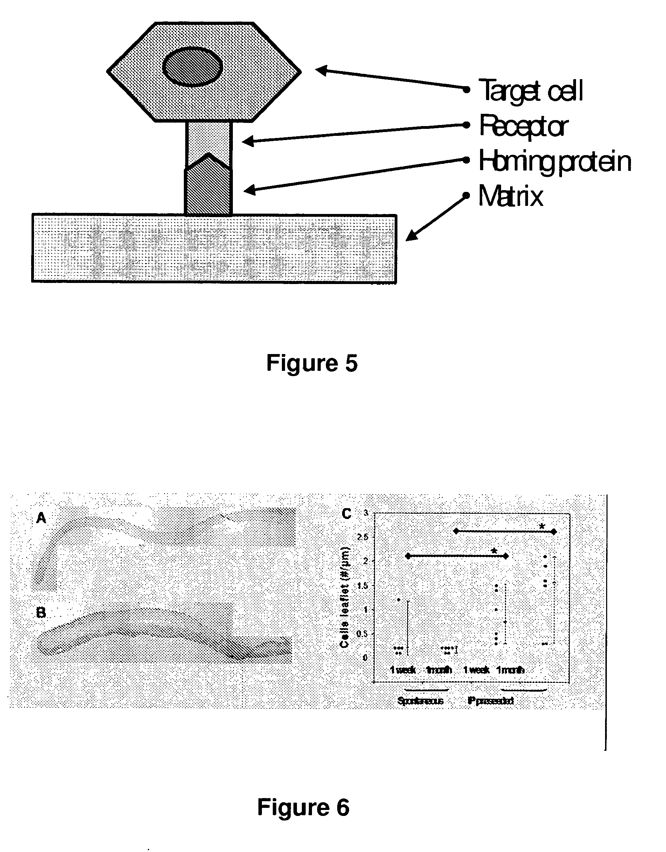

According to one aspect of the present invention, the presence of one or more homing factors ensures the binding of one or more particular cell types to a matrix. Thus, in the context of tissue engineering, more particularly tissue engineering of of vascular or lymphatic organs or other vessels such as the urethra, where the matrix is constantly in contact with fluid, such as the blood or lymph fluid and the cells therein, the presence of homing agents alone can suffice to ensure the population of the matrix with the appropriate cells. The present invention demonstrates that homing factors can be used to physically and specifically link appropriate cells to a matrix of choice (Figure 5). Thus a particular embodiment of the present invention relates to scaffolds comprising homing factors. Most particularly, the present invention relates to scaffolds which comprise on their surface only one or more homing factors, and no other proteins involved in chemo-attraction, mobilization, etc.. Not only will the homing factors sufficiently ensure the binding of the relevant cells to the matrix, but the absence of other molecules which affect cell attraction and/or mobilization avoids potentially important negative side-effects such as vascularization of the graft.

However, it is also envisaged within the present invention that a combination of homing factors and other bio-active molecules is used for the coating of the matrix making up the scaffold of the present invention.

Thus, according to a further embodiment of the invention, the matrix further comprises, in addition to the one or more homing factor(s), one or more other factors which facilitate the binding of appropriate cells to a matrix. Such other factors include mobilisation agents, chemoattractive agents and facilitating factors.

"Mobilisation agents" in the context of the present invention, are agents that mobilise cells, such as stem cells from the place in the body where they originate. More particularly, mobilisation agents are used to increase the amount of stem cells in the blood. A particular example of a mobilisation agent is granulocyte colony stimulating factor or G-CSF. This naturally occurring factor has low toxicity and synergistic effect when combined with other haematopoietic growth factors. Other mobilisation agents are adenosine, granulocyte monocyte colony stimulating factor (GM-CSF), stem cell factor (SCF), interleukine-1 (IL1), IL3, IL7, IL11 and IL12 (Fu & Liesveld (2000) Blood Rev. 14, 205-218.). The use of mobilisation factors in addition to the homing factors according to the present invention is of particular interest in those tools and methods which envisage the seeding of the matrix *in vivo.* The mobilisation agent is optionally included in the matrix but can also be administered to the body before implantation of the scaffold.

Additionally or alternatively, the present invention envisages the use of one or more chemoattractive factors together with the homing factor(s) and optionally mobilisation agent(s) described above. "Chemoattractant" or "chemotactic compounds" as used herein are compounds capable of attracting cells. In general, they have an effect on cells when present in a gradient. Possible chemoattractive agents for stem cells are insulin-like growth factor (IGF) and vascular endothelial growth factor (VEGF) (Young et al. (1999) Clin. Exp. Metastasis 17, 881-888).

Additionally or alternatively, the present invention envisages the use of one or more facilitating agents together with the homing factor(s) and the optional additional factors described above. The "facilitating factors" are factors which facilitate or strengthen the binding of cells to the homing proteins or factors of the present invention. Examples of such facilitation proteins include soluble collagen, albumin, fibrinogen or fibronectin. The facilitating proteins can be coated on the matrix together with the homing factor or can be coated as a separate layer.

Thus, the present invention relates to a matrix for use in the tissue engineering of vessels or other implantable scaffolds, such as blood vessel or heart valves, comprising a matrix with one or more homing factors. More particularly, the invention relates to a matrix comprising SDF-1 and/or SCF designed to replace deficient, diseases or disordered heart valves and for in vivo recellularisation. The SDF-1 loaded matrix or scaffold may comprise additional homing agents and may furthermore comprise one or more mobilisation agent for instance a mobilisation agent selected of the group consisting of granulocyte colony stimulating factor (G-CSF), adenosine, granulocyte monocyte colony stimulating factor (GM-CSF), stem cell factor (SCF), interleukine-1 (IL1), IL3, IL7, IL11 and IL12 or a combination thereof. The matrix may further comprise one or more chemoattractive agent for instance chemoattractive agent selected of the group consisting of insulin-like growth factor (IGF), vascular endothelial growth factor (VEGF); Placental growth factor (PLGF) or a combination thereof.

According to one embodiment, the cellularisation paradigm of the present invention comprises three steps (Figure 2). Optionally, an initial stem cell mobilisation step, secondly the implantation of a scaffold (e.g. vessel or valve) coated with homing factors and optionally the release of chemoattractive agent by the scaffold. According to one particular embodiment such a scaffold is a vessel or valve for use in the cardiovascular system. In this embodiment however, since the valve or blood vessel grafts are implanted into the diseased valve or blood vessel position and are thus in immediate contact with the blood, it is therefore sufficient to implant the matrix with the homing factors, optionally in combination with chemoattractive agents.

The heart valve construct prepared for endogenous cell attachment according to the present invention can be implanted by a standard implantation procedure such as, but not limited to, the one decribed hereafter. Heart valves are implanted in orthotopic or heterotopic position with or without complete or partial removal of the native valve. Classical implantation techniques are used as describedherein or the implantation can involve minimal invasive, endovascular or percutaneous (see below) approaches. Implantation methods for vascular grafts are similarly known in the art.

The technology of the invention can also be applied to percutaneously implantable valve prosthesis by a method decribed in documents such as, but not limited to, EP 1152780A1 and WO 0045874A1. These patent applications describe a device for implantation of a heart valve via a percutaneous route comprised of a peripheral deployment balloon and a central axial blood flow pump, and are particularly suited for grafts which do not require in vitro seeding or maturation.

The present invention thus relates to a matrix for use in the engineering of organ or tissue scaffolds, more particularly scaffolds of organs and/or tissues which are subject to shear stress, such as scaffolds of the cardiovascular or lymphatic system or scaffolds of the urinary tract. Most particularly the present invention relates to heart valve and blood vessel tissue scaffolds. According to a particular embodiment of the present invention, the scaffold comprising the matrix with the homing factor(s) according to the present invention is modelled to the shape of the implantable device to be used, and thus corresponds to a pre-fabricated organ, valve or vessel. The scaffold should be bio-compatible and either surgically or percutaneously implantable. The present invention envisages both stented and stentless scaffolds.

Examples of suitable matrixes for use in the context of the present invention include artificially produced and molded scaffolds, which can be of synthetic polymer or of biological material such as collagen or fibrin and natural scaffolds such as accellular root material or cross-linked natural material such as pericardium (Figure 1). A biological matrix includes a matrix obtained de novo from biological material (such as human fibrin gel) as well as a matrix which retains the structure of origin, such as, but not limited to acellularized aortic root. The biological material can be autologous (from the patient in which the device is to be implanted), homologous (e.g. from human material if the implant is to be implanted into a human) or of heterologous origin (e.g. bovine, porcine or ovine in the case of a human implant). The biological matrix is either fresh or treated in such a way so as not to compromise the growing of cells on the surface of the matrix or the flexibility of the matrix (e.g. cryopreservation, UV radiation, photooxidation). Synthetic grafts can be made up of materials such as polyester, expanded polytetraflourethylene (ePTFE) and other composite materials as known in the art. Particularly, according to the present invention the synthetic matrix is a biocompatible and biodegradable material such as polyglycolic acid meshes and polyhydroxyalkanoate, a bacterium-derived thermoplastic polyester. Synthetic devices can alternatively be made up of materials such as polyester, expanded polytetraflourethylene (ePTFE) and other composite materials as known in the art.

According to a particular embodiment of the present invention, the matrix is non-biodegradable and thus durable, to allow prolonged presence and functioning within the body. Matrices derived from bovine pericardium from which cells have been removed (such as Veritas™ Collagen Matrix or SynerGraft™) are envisaged within the context of the present invention.

The "coating" of the homing factor(s) on the matrix according to the present invention can be done in a variety of ways. Among the coating procedures three coating processes are particularly suitable; for homing factors which naturally bind to the matrix, an impregnation procedure can be used. This binding will be both homing factor- and matrix-dependent. Impregnation involves an incubation of the matrix in a solution comprised of the homing factor in an appropriate solvent such as but not limited to phosphate buffered saline. This solution is applicable for both precoated devices and a coating kit allowing pre-implantation coating in the operation room.

Alternatively, according to the present invention, a co-coating with either soluble collagen, albumin, fibrinogen or fibronectin is performed. This is particularly suitable when homing factors are used which interact with these proteins. This method includes a pre-coating with an extracellular matrix protein. This precoating is based on interactions with cross-linked native proteins in the matrix and their natural counterparts. In a second step the homing factors will then bind to these added extracellular matrix proteins. Here again, this procedure can be applied before shipping of the implantable devices as well as a kit-format allowing the coating in the OR.

Alternatively, a chemical cross-linking with or without a spacer-molecule can be ensured between the matrix and the homing factor. The spacer can be a permanent or bio-degradable linker, as once the cells have been attached, the presence of the homing factors is less critical. The factor can be cross-linked immediately to the matrix provided that it remains functional. Another embodiment involves a biochemical cross-linking with an interspaced linker arm. The specific architecture of this linker arm allows control of the biodegradability of the cross-linking and as such the pharmacokinetics of the added homing factor. Different methods for this cross-linking have been described in the art. A particularly useful paradigm is the use of a photochemical cross-linking as decribed in EP 0820483B1, but different methods of cross-linking are envisaged (e.g. methods described in patent publications EP0991944B1, EP1035879B1 and W00159455A2).

According to a particular embodiment of the invention one or more homing factors and/or chemoattractant and mobilisation factors are present on the matrix in the form of a fusion protein. A fusion protein can be obtained by recombinant technology. The fusion protein is then specifically chosen for interaction with the matrix, as such the fusion protein comprises a homing moiety as well as a matrix interaction moiety. Generally a fusion protein is produced by a host organism which has been genetically altered by insertion of a gene, comprised of the combination of 2 genes each encoding a specific protein. This allows the combination of any of the aforementioned homing factors with a protein interacting with the cross-linked matrix. Here again, the latter protein in the fusion protein construct can be the full or partial polypeptide of molecules such as, but not limited to, collagen, fibrinogen or fibronectin. The fusion protein is in general selected for its specific cell homing and matrix binding properties. The fusion protein can be applied to the implantable device either before shipping or in kit-format immediately before implantation into the recipient.

Precautions need to be taken in order to prevent inactivation of the protein by any subsequent treatment of the valve (i.e. sterilization). A sterilization technique which does not significantly alter the bioactivity of the mobilisation agents, chemoatractive agent or homing agents is preferable. Adequate sterilisation conditions which can preserve the biological activity of the mobilisation agents, chemoatractive agent or homing agents, are present in the art such as sterilization of the loaded matrix with e.g. a low dose gamma radiation or ethylene oxide. Particularly suitable methods of sterilization are ethylene oxide at a temperature selected from within the range of 37 to 63 °C or radiation with about 1 to about 3 mRad of gamma radiation or electron beam radiation. If the bioactive agent is a protein or peptide, biological activity can be optimized during gamma radiation sterilization by including in the formulation 1) an extraneous protein, for example albumin or gelatin; and 2) a free radical scavenger (antioxidant), for example propyl gallate, 3-tert-butyl-4-hydroxyanisole (BHA) or ascorbic acid, in amounts effective to retard radiation-induced degradation of the biologically active peptide. The sterilization is preferably conducted at low temperature, for example -70°C.

According to another aspect the present invention relates to methods of treating a patient having a diseased or damaged tissue, vessel or organ, such as but not limited to a diseased or damaged blood vessel or heart valve, which method includes implanting in said patient the scaffold of the present invention coated with one or more homing factors.

According to a particular embodiment, the scaffold is implanted for seeding *in vivo*. Alternatively however seeding *in vitro* is also envisaged. The *in vitro* seeding can take place in a bioreactor. Bioreactors suitable in the context of the present invention are known in the art and include those described by Hoerstrup et al. (2002, Tissue Engineering 8(5): 863-870).

Particular embodiments of the homing factors used according to the present invention and the cells attached therewith are illustrated in Figure 4. It will be apparent to those skilled in the art that various combinations can be made of homing factors. Furthermore, various modifications and variations in the manufacturing and use of the scaffolds of the present invention and in construction of the system and method are also envisaged.

The scaffolds of the present invention provide particular advantages over the prior art scaffolds, which entail a number of risks as illustrated in Figure 3. A first risk is an exposure of the patient's cells to xenogeneic pathogens (e.g. prions, viruses and others). The adventitious agent risk is introduced via either proteolytic enzymes or culturing media. Although these factors are typical for the *in vitro* phase of heart valve tissue engineering the risk is not limited to these routes of infection. Other routes of infection are xenogeneic matrix materials or cross infection between patient's cells when treated within the same facility. Viral infections pose some specific risks, many of which are dependent on the strain of infection, such as post implantation patient infection, mutagenesis by genomic insertion of the viral DNA and proto-oncogene function of viral proteins possibly inducing immortalisation. The second family of risks are the unphysiological cellular environment factor, to which the cells are exposed under in vitro conditions. An example is the potential DNA-damage induced by unphysiological oxygen tension inducing oxidative stress. The conclusion is that each of these factors need to be tested because the cells are "self" and will be accepted by the patients immune system disregarding their potentially inducted damage.

### BRIEF DESCRIPTION OF THE DRAWINGS

The following Figures illustrate the invention but are not to be interpreted as a limation of the invention to the specific embodiments described therein.
- Figure. 1:: Schematic view showing a general overview of the scaffold choices according to a particular embodiment of the present invention. A first group are molded scaffolds. These use either biological or non- biological thermoplastics to create a valve scaffold. In general, this is achieved using a cast in which the thermoplastic is allowed to harden or by a process known as electrospinning. Another option is to use proteins such as collagen or fibrin to create such a valve. These construct are made out of a natural protein, obtained from e.g. a patient, are of allogeneic or xenogeneic origin or recombinant. These proteins are then allowed to interact with each other in a valve mold. The second group are natural scaffolds, i.e. either allogeneic or xenogeneic biological valves. Two major classes can be distinguished: (1) acellularised aortic roots and (2) cross-linked prosthesis.
- Figure 2:: Schematic diagram showing the complete tissue engineering paradigm as implemented in heart valve tissue engineering according to one embodiment of the invention. A valve construct is made by seeding of appropriate cells on an appropriately chosen scaffold. These cells can be endothelial cells, fibroblasts or valve interstitial cells. The in vitro created valve construct is then placed into a bioreactor for a certain period of time to mature the construct, while accustoming the cells to gradually increasing flow and pressure. The mature construct is then generally after some weeks implanted into the recipient where it can be subjected to in vivo remodelling.
- Figure 3:: Schematic view showing the risks of the complete paradigm in heart valve tissue engineering according to an embodiment of the present invention.
- Figure 4:: Schematic view showing homing proteins and respective receptors present on specific cell types according to one embodiment of the invention. The P1 or P2 epitope of fibrinogen interacts with the mac1 integrin expressed by macrophages. Stem cell factor (SCF) binds to the protein tyrosine kinase receptor (c-kit or CD117) of "mesenchymal" stem cells (MSC). Homing of haematopoietic stem cells (HSC) can be achieved by different interactions. This preferentially is achieved by stromal derived factor 1 (SDF-1) which binds to its receptor CXCR4. HSC's also attach to fibronectin (FN) by means of very late antigen (VLA) 4 or 5, additionally VLA-4 is also binding vascular cell adhesion molecule 1 or VCAM-1. The latter binding can be enhanced by the pleiotropic protease inhibitor a2-macroplobuline (a2-MG).
- Figure 5:: Schematic conformation of a scaffold of the present invention after cell seeding according to one aspect of the invention. A homing protein on the matrix interacts with a receptor of an attracted cell. The specificity of cell binding is determined by the appropriate choice of homing protein.
- Figure 6:: Example of a spontaneous seeded leaflet (A) and a preseeded IP (B) according to one aspect of the invention. Panel C shows the recellularisation (total cell count/ leaflet length) of both spontaneous seeded and IP preseeded leaflets at 1 week and 1 month *:p<0.05.
- Figure 7:: Histology. Graph A:medium value of overgrowth of both spontaneous seeded and IP preseeded leaflets at 1 week and one month. Graph B: median surface of newly deposited matrix upon the bovine photooxidised pericardium. Graph C:median value of the leaflet length measured from the surface to the tip. *:p<0.05

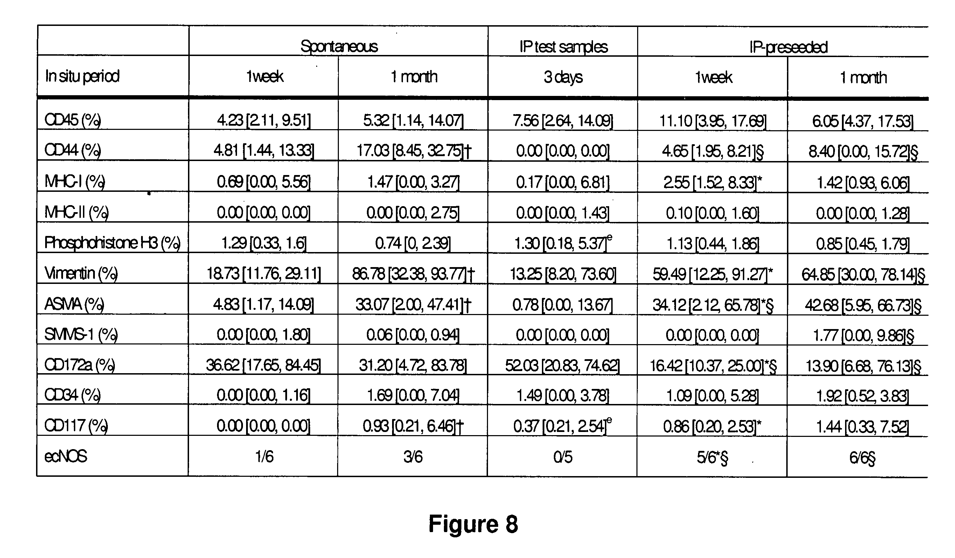

- Figure 8:: Characterization of implanted valves using antibodies to cellular markers as described herein in Example 1. Data are presented as median [95% CI]. * indicates significant difference between the 1 week groups; † indicates significant difference between either both control groups or between both IP seeded groups; ‡ indicates difference between both 1 month groups; § indicates significantly different from I P test samples, e indicates that no statistical analysis could be performed because n < 6.

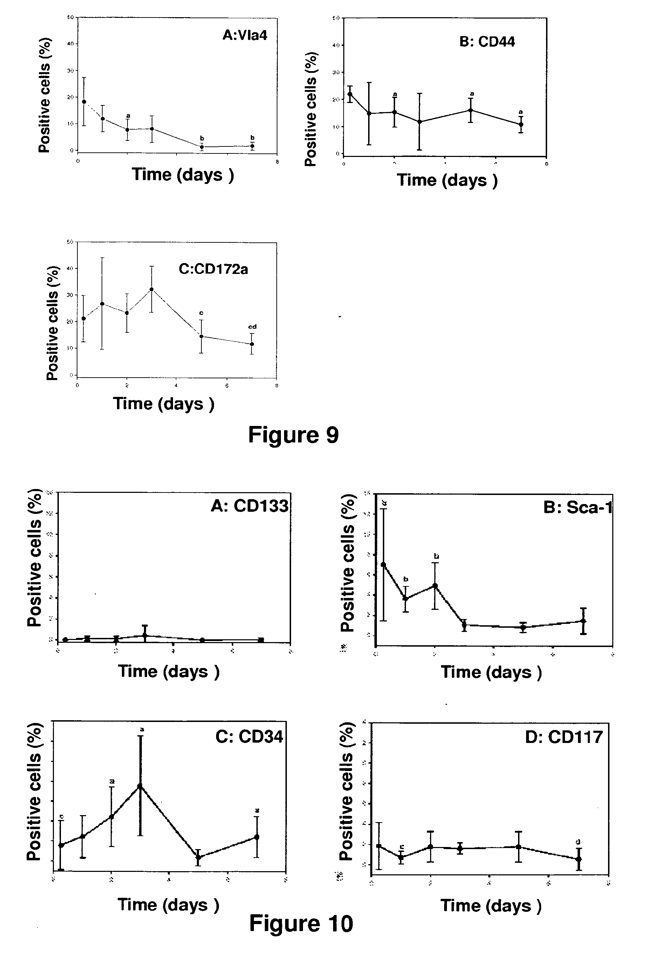

- Figure 9:: Percentage of (A) VLA-4+ (B) CD44+ and (C) CD172a+ cells present in the material during the different stages of the FBR. Dots and error bars represent average ± standard deviation. a) significantly different from 6 hours (p<0.05); b) significantly different from 6 hours, 1, 2 and 3 days (p<0.05); c) significantly different from 3 days (p<0.05); d) significantly different from 2 days (p<0.05). For CD172a, the 6 hours data are excluded from the statistical analysis because of 2 missing rat data. Cell binding and homing capacity is high, directly after implantation and is generally decreasing afterwards, except for a significant peak in CD172a+ cells at day 3.
- Figure 10:: Percentage of (A) CD133+ and (B) Sca-1+ primitive stem cells and the percentage of (C) CD34+ and (D) CD117+ progenitors present in the material during the different stages of the FBR. Dots and error bars represent average ± standard deviation. a) significantly different from 5 days (p<0.05); b) significantly different from 3, 5 and 7 days (p<0.05); c) significantly different from 3 days (p<0.05); d) significantly different from 2 days (p<0.05). As can be seen, Sca-1+ primitive stem cells have a peak in their presence at 6 hours after implantation, while CD34+ and CD117+ progenitor cells have a peak in their presence at 2 and 3 days after implantation.

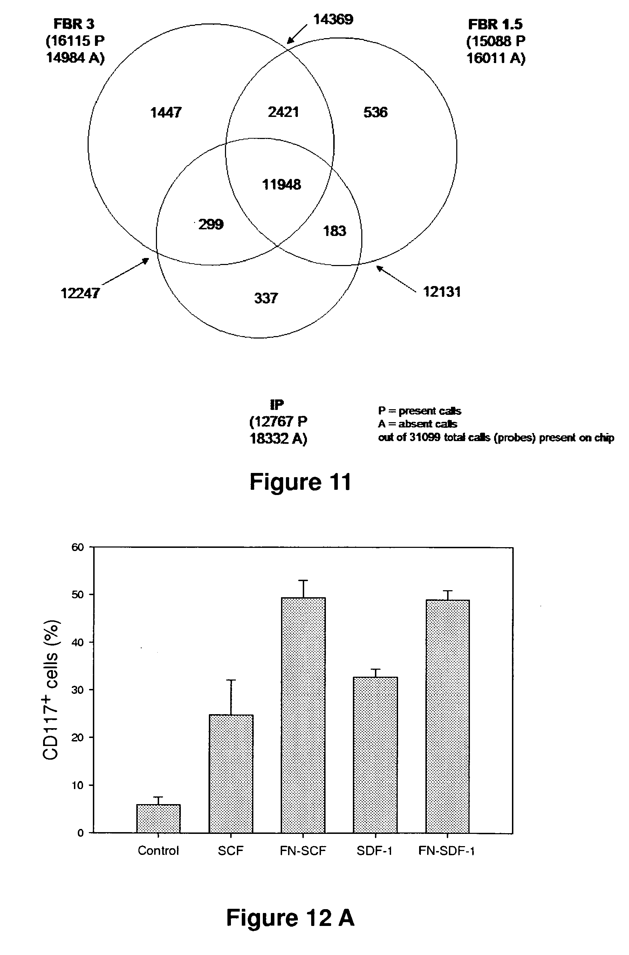

- Figure 11:: Result obtained from the microarray assessed gene expression profiles of intraperitoneal implants after 1.5 and 3 days and peritoneal macrophage (IP) according to one aspect of the invention.

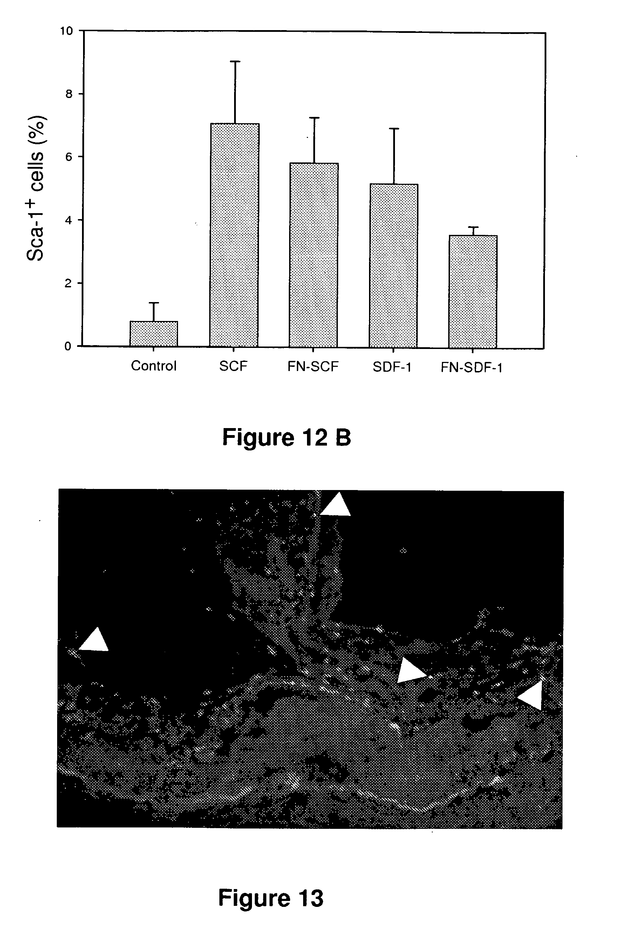

- Figure 12:: Results of the CD117 (a) and Sca-1 (b) positive cell fraction present on the controls and SDF-1 and SCF impregnated (with or without FN precoating) carotid artery grafts. Asterisk indicates significant difference from control (n=6 in each group) and # indicates difference from SDF-group
- Figure 13:: Presence of CD34+ cells (haematopoietic stem/progenitor cells) on patches implanted in sheep arteries.

### EXAMPLES

### Example 1: Engineering a valve by IP implantation of a scaffold

In order to obtain a seeded matrix for identifying the key factors involved in the adhesion of appropriate cells, an immature foreign body reaction was used to repopulate a cross-linked biological matrix, namely photooxidised bovine pericardium. Such a type of matrix ensures durability by itself. In sheep, a three day intraperitoneal (IP) implanted scaffold or patch becomes covered with blast-like cells with a mesenchymal origin and immature differentiation which could and do normally differentiate into a myofibroblast phenotype. More particularly, it was found that these cells were positive for vimentin but negative for α smooth muscle actin and heavy chain myosin (see Table 1).

**Table 1: comparison between 3 day IP seeding in sheep and rats.**

| | CD44 | CD45 | CD172a | Vimentin | ASMA |
|---|---|---|---|---|---|
| Sheep (n=10) | 0.0 [0.0, 0.0] | 7.6 [2.2, 16.5 | 52.0 [19.9, 82.8] | 13.2 [7.5, 89.0] | 0.8 [0.0, 15.4] |
| Rat (n=6) | 9.4 [1.6, 25.2]* | 12.7 [11.3, 29.4] | 32.1 [18.4, 41.8] | 26.5 [20.5, 29.4] | 0.2 [0.0, 1.2] |

| | SMMS-1 | PPH3 | CD34 | CD117 | |
|---|---|---|---|---|---|
| Sheep (n=10) | 0.0 [0.0, 0.0] | 1.3 [0.2, 5.4] | 1.5 [0.0, 7.2] | 0.3 [0.2, 2.5] | |
| Rat (n=6) | 0.0 [0.0, 0.0] | 5.9 [1.7, 7.8] | 7.3 [1.0, 15.0]* | 1.7 [0.8, 2.3] | |

| | | | | | |
|---|---|---|---|---|---|
| * indicates significant difference between sheep and rat (p<0.05) | | | | | |

It was found that a stable matrix with proven durability could be obtained to which differentiates into a different phenotype within a few days.
A valve was constructed out of the sheep IP-matured material and implanted into the pulmonary artery of sheep, and assessed for functionality. 24 valves were implanted (12 unseeded controls and 12 intraperitoneally seeded valves). In each of the two groups 2 time points were studied 1 week (n = 6 per group) and 1 month (n = 6 per group) post implantation in the pulmonary artery. Valve function was assessed by echocardiography. No abnormalities were observed. No major thrombus formation was seen in the intraperitoneally seeded grafts whereas 1 was found in the control group.

**Table 2: Sheep and echocardiography data**

| | Control | | I P preseeded | |
|---|---|---|---|---|
| | 1 week | 1 month | 1 week | 1 month |
| Number | 6 | 6 | 6 | 6 |
| Age | 737 [362, 764] | 706 [368, 722] | 575 [452, 773] | 653 [575, 719] |
| Weight | 68 [64, 69] | 66 [43, 72] | 65 [59, 74] | 68 [66, 77] |
| Peak gradient (mmHg) | 9 [8, 25] | 11 [7, 20] | 11 [9, 39] | 10 [8, 39] |
| Normal function | 6/6 | 6/6 | 6/6 | 5/6 |
| PI | 1/4 [0/4, 2/4] | 1 /4 [1/4, 1/4] | 1/4 [0/4, 3/4] | 1 /4 [1/4, 2/4] |
| Thrombus | 0/6 | 1/6 | 0/6 | 0/6 |

Using histological staining it was found that there was a marked difference in cellularisation between controls and intraperitoneally seeded valve constructs. In contrast to the controls, a significant deposition of cells and new matrix was observed on the intraperitonally seeded valve, which also clearly showed a repopulation of the original matrix (see Figures 6 and 7). 7µm cryosections of the explanted valve and control samples were immunofluorescently stained for CD44 (clone BAT 31A, VMRD Inc.), CD45 (clone 1.11.32, Serotec), CD172a (clone DH59B, VMRD Inc.), Vimentin (clone V9, DAKO), ASMA (clone 1 A4, DAKO), SMMS-1 (clone SMMS-1, DAKO), Phosphohistone H3 (polyclonal, CAMPRO scientific), CD117 (polyclonal, ABCAM), ecNOS (clone 3, BD Biosciences), MHC-I (clone H58A, VMRD, Inc), MHC-II (clone TH14B, VMRD, Inc and CD34 (clone QBEnd 10, DAKO).
[CD44: H-CAM, cell surface molecule binding hyaluronic acid; CD45: leucocyte common antigen; CD172a: is a marker for monocytes and stem cells; Vimentin: is a marker for mesenchymal cells; Alpha smooth muscle actin (ASMA): is a marker for myofibroblasts and smooth muscle cells; Heavy chain myosin (SMMS-1): is a marker for smooth muscle cells; Phosphohistone H3: is a marker for mitosis; CD117 is a marker for stem cells; ecNOS: is a marker for endothelium; MHC-I and MHC-II are markers for immune respons; CD34: is a marker for progenitor cells]
Cells which are present in and on the implanted matrix material, in one complete leaflet section were counted. Spontaneous seeded control had 3753 [995, 17254] and 3345 [1562, 4298] cell per section at 1 week and 1 month after implantation in the pulmonary position, respectively. IP preseeded valves, on the other hand, had 12126 [4571, 28216] and 20404 [4723, 32084] cells per section at 1 week and 1 month respectively. This four to sevenfold increase in cell count between spontaneous and IP preseeded valves was significant.
The percentages of positive cells found in the valve sections are summarized in the Table in Figure 8. Data of all groups and stainings are represented as median values and the 95% confidence interval. Since these are percentages, only the fraction of positive cells is shown, keeping in mind the large differences in total cells observed, large differences in the absolute cell type count are apparent.
These findings clearly illustrate the potential of these cells to revitalise the stabilised biological matrix with myofibroblasts. Furthermore the 1 month implants already showed signs of spontaneous reendothelialisation. The process of recellularisation appears to be self-limiting, since the amount of newly deposited material is not continuously increasing and is getting covered by endothelium. This prevents new cells from adhering and contributing to the recellularisation process.
Although stabilised biological matrix has been used, the cells obtained by peritoneal seeding are able to modify this matrix as well as to deposit their own.
This biohybrid valve constructed out of xenogeneic matrix material and autologous cells combines the reliability of the matrix with the viability of the cells. The cells are of mesenchymal origin and can differentiate into the appropriate phenotype, myofibroblast, for cell repopulation. The present study demonstrates that the repopulation although mediated or initiated by macrophages, is (haematopoietic) stem cell derived.

### Example 2: Stem cell attraction to intraperitoneal matrix

Matrix material was introduced intraperitoneally in rats and the type of cells attracted was investigated.

### Animals

Male Wistar rats (n=36; 380-400g) were selected. Access to food was given ad libitum. All animals were cared for in accordance with the 'Guide for the Care and Use of Laboratory Animals' (NIH publication 85-23, revised 1985). The study was approved by the local Ethics Committee.

### Procedure

Anaesthesia was induced with 4% isoflurane in 100% oxygen 1 I/min for 5 minutes and maintained with 2% isoflurane in 100% oxygen 0.5 I/min during the surgical procedure taking approximately 20 min. After shaving and disinfecting, a pararectal incision of approximately 1.5 cm was made through the skin, abdominal muscles and peritoneum. The stainless steel cage containing the matrix material was inserted into the abdominal cavity and fixed to the abdominal wall with transabdominal sutures (Ticron 3-0). The peritoneum and abdominal muscles were closed with a running suture (Ticron 3-0) and the skin was sutured intradermally (Ticron 3-0) to avoid opening of the wound by grooming. Following the surgical procedure, the anaesthesia was discontinued. After approximately 5 min, the animals regained conscience and were placed in individual cages.

### Conditions

Retrieval of the matrix materials was performed at different time points. The different retrieval times were 6 hours, 1, 2, 3, 5, and 7 days after implantation, depending on the group to which the animal was assigned. For this purpose, the animals were re-anaesthetised, the wound reopened and the cage removed.

### Material

The retrieved matrix material was embedded in Tissue Freezing medium (Leica - Van Hopplynus instruments, Brussels, Belgium), snap frozen in liquid nitrogen and stored at -80°C. Cryosectioning was performed on a Microm HM500 OM cryostat (Prosan, Merelbeke, Belgium). The 7 µm sections were placed onto poly-L-lysine coated slides and stored at -20°C until staining.
Prior to staining, the material was fixed in ice-cold acetone for 10 min. Subsequently the matrix sections were immunohistochemically stained with antibodies. Primary antibodies were detected with FITC-conjugated secondary antibodies. Pictures were taken at room temperature using an Axioplan 2 imaging microscope with a Zeiss Axiocam MRc5 camera (Zeiss; Zaventem, Belgium). The objective lenses used were Plan-NEOFLUAR 1 x/0.025, Plan-APOCHROMAT 10x/0.45 and 20x/0.75. Image analysis was performed with Axiovision Rel. 4.4.
To study the cellular phenotypes (immunohistochemistry) in detail, they are grouped according to function or specificity. The cell density and proliferation were studies at 6 different time points (n=6 per time point) after intraperitoneal implantation.

**Table 3: Cell count and in situ proliferation**

| | Implantation time | | | | | |
|---|---|---|---|---|---|---|
| | 6h | 1d | 2d | 3d | 5d | 7d |
| Cell count (cells/mm) | 128 ± 55^{b} | 397 ± 181^{ab} | 377 ± 148^{ab} | 722 ± 489^{a} | 743 ± 392^{a} | 569 ± 331^{a} |
| PPH-H3 (%) | 0.708 ± 1.229^{c} | 0.601 ± 1.104^{c} | 0.869 ± 1.869^{c} | 5.44 ± 2.26 | 1.68 ± 1.94^{c} | 0.505 ± 0.871 ^{c} |

| | | | | | | |
|---|---|---|---|---|---|---|
| Data are represented as average ± standard deviation. a significant difference from 6h (p<0.05); b significant difference from 5d (p<0.05); c significant difference from 3d (p<0.05). | | | | | | |

Table 3 shows that cells are present on the completely acellular implanted material from 6 hours after the intraperitoneal implantation onwards. In the implants, a general increase in cell number over time can be seen, becoming significant from day 1 onwards. At 7 days post implantation, a more than 4-fold increase in cell number is observed. The marker used to assess in situ cell proliferation, phosphohistone H3 (PPH-H3), shows a significant peak of approximately 5% in in situ cell proliferation at day 3 post implantation. Except for the presence of the peak in cell proliferation at day 3, these data are supportive for a neogenesis of tissue by cell influx rather then by cellular division. It is only at day 3 that the cellular proliferation seems to be contributing to the increase in cellularity.
Cell binding and homing capacity were assessed with antibodies to VLA-4 (CD49d), CD44 and CD172a or signal regulatory protein alpha (SIRPα). Figure 9 shows that VLA4+ cells were clearly present in the first stage of the FBR comprising roughly 20% of the cell population and that this fraction decreases significantly to approximate absence after 5 days of intraperitoneal implantation. CD44+ cells show a similar starting presence but significantly decrease at days 2, 5 and 7, as compared to the 6 hours measurements, but maintaining their presence at about 10% until day 7. The CD172a data show a similar pattern of presence of these cells, situated within the same order of magnitude as the CD44 data, except for the fact that the decrease is not significantly present and that a small but statistically significant peak is situated at day 3. VLA-4 (CD49d), found on T-cells, B-cells, thymocytes, CD34+ hematopoietic stem cells and endothelial cells, is an integrin molecule which binds vascular cellular adhesion molecule-1 (VCAM-1) on the marrow stroma and is involved in the homing of stem and progenitor cells to the marrow stroma (Krause et al. (1996) Blood 87, 1-13). VLA-4 also mediates attachment of hematopoietic progenitor cells to fibronectin ( Levesque & (1999) Exp. Hematol. 27, 579-586). CD44, an adhesion molecule on leukocytes, hematopoietic progenitor cells (Netelenbos et al. (2002) J Leukoc. Biol. 72, 353-362) and mesenchymal stem cells (Rombouts & Ploemacher(2003) Leukemia 17, 160-170), has been shown to mediate cell-cell and cell-ECM interactions, to play a role in leukocyte trafficking to sites of inflammation and to costimulate lymphocyte activation and tissue infiltration (Wu et al. (2005) Cell Res. 15, 483-494). Furthermore, interactions of the cell surface proteoglycan CD44 with the extracellular matrix glycosaminoglycan hyaluronan (HA) are key events in inflammation (Levesque et al. cited above). The elevated levels of both VLA-4+ and CD44+ cell fractions immediately after implantation are clearly indicative of an increased cell binding and homing capacity mediated by these molecules which decreases significantly during the later stages when the cells start to differentiate, which is also observed in the absolute cell numbers.
Most importantly, 4 markers predominantly expressed by stem/progenitor cells were studied: CD133, stem cell antigen-1 (Sca-1), CD34 and CD117 (c-kit) (see Figure 10). The CD133+ cell fraction, representing the most primitive stem cell studied (Gehling et al. (2000) Blood 95, 3106-3112) was very small, reaching its maximum of 2.3% in 1 rat after 3 days of implantation. Sca-1 is known to be presented by primitive hematopoietic and mesenchymal stem cells. During the early phase of the intraperitoneal implantation approximately 7% of these cells were found. CD34 and c-kit (CD117) are both markers for circulating hematopoietic stem and progenitor cells (Okamoto et al. (2005) Blood 105, 2757-2763). C-kit is also expressed on mesenchymal stem cells. The temporal profile of CD34+ cells shows a gradual increase in the fraction of these cells found on the implant material, reaching a significant peak value of about 5-8% at days 2 and 3. Remarkable is the very rapid return to low levels of CD34+ cells already apparent at day 5, followed again by a significant increase towards day 7. The c-kit pattern shows a significant elevated level of approximately 2% at days 2 and 3. The expression of CD133, a transmembrane cell surface antigen, is restricted to a subset of the CD34+ stem and progenitor cells (Buhring et al. (1999) Ann. N. Y. Acad. Sci. 872, 25-38) and to endothelial precursor cells (Gehling et al. 2000, cited above). Additionally, it is expressed by a small portion of approximately 0.2% of the CD34- cells (Gallacher et al., cited above). CD34+CD133+ cells are enriched in primitive and myeloid progenitor cells, whereas CD34+CD133- cells mainly consist of B-cell and late erythroid progenitors (Buhring et al. 1999, cited above). The CD133 antigen is sporadically present in theimplanted patches, suggesting a very limited contribution of these primitive cells in the formation of new tissue in FBR. Sca-1 is expressed on multipotent primitive hematopoietic stem cells in bone marrow and in peripheral blood, as well as on mesenchymal stem cells. Sca-1+ cells are more primitive than Sca-1- cells and respond better to a combination of hematopoietic factors, including SCF and stromal cells (Okada et al. (1992) Blood 80, 3044-3050; Rombouts and Ploemacher cited above; Spangrude et al. (1991) Blood 78, 1395-1402.). Interestingly, a rather large fraction of these cells was observed immediately after the implantation and a gradual decrease in the later stages. A peak in absolute cell number was observed 2 days after implantation. Taken together, the findings clearly indicate that these cells are a major and early contributor to the FBR reaction.
Both CD34 and CD117 were used as markers for more committed stem and progenitor cells as compared to CD133 and Sca-1. The marker CD34, a single chain membrane protein, indicates the presence of hematopoietic stem/progenitor cells, endothelial precursor cells and capillary endothelial cells. C-kit (CD117), a member of the receptor tyrosine kinase family and the receptor of stem cell factor (SCF), is expressed on hematopoietic primitive stem cells and committed progenitor cells (Okada et al. cited above), on circulating immature cells (Taguchi et al. (2004) Circulation 109, 2972-2975) and on mesenchymal stem cells (Rombouts and Ploemacher cited above). These results showed a temporary elevated level of both CD34+ as c-kit+ cells at 2 and 3 days of implantation, which was especially pronounced in the CD34+ cell fractions. These cells showed a clear peak at day 3 approximating the initial fraction of Sca-1+ cells. These findings can be explained by either differentiation of the Sca-1+ cells into the more committed c-kit+ or CD34+ cells or by temporary recruitment of these types of cells from the bloodstream through signaling factors released from other cells present in the early phase FBR, probably the macrophages. Since the absolute number of CD34+ cells at its peak largely overshoots the absolute Sca-1 cell count, the latter explanation is more acceptable.
By present invention it was surprisingly found in a rat intraperitoneal implantation model that that during immature foreign body reaction stem cells and progenitor cells are attracted to the tissue and are actively involved in the repopulation of the matrix with (myo)fibroblasts.

### Example 3: Specific gene expression in tissue neogenesis.

As mentioned before , the tissue neogenesis was studied as it occurs in the FBR in adult animal models, because it is able to produce laminar tissue with a cellular component similar to vascular structures such as heart valves (Butler et al. 2001 a cited above; Butler et al. 2001 b cited above). Unfortunately the mature tissue is not an ideal solution since it would require the construction of a valve prosthesis in the operation room, a method prone to variation of the valve quality (Grabenwoger et al. (2000) J Heart Valve Dis 9, 104-109). Nevertheless the tissue neogenesis *in se* is an interesting feature because it contains all the components, that is, the cells (example 2), new extracellular matrix, signaling molecules and homing proteins, necessary to construct a new tissue. Homing proteins, molecules responsible for the physical linkage of the cells to the extracellular matrix, were identified.
Similar to example 2, photooxidised bovine pericardium, a completely acellular and cross-linked matrix, was suspended in a stainless steel cage and implanted into the abdominal cavity of Wistar rats. Two different implantation periods (1.5d and 3d) have been studied with 2 rats in each group. The implants were retrieved after 1.5d or 3d, depending on the group to which the animal was assigned. For this purpose the animals were re-anaesthetised, the wound reopened and the cage removed. Upon retrieval the matrix patch was immediately put in RNAlater RNA Stabilization reagent (Qiagen) until RNA extraction.
The background gene expression, that is the gene expression of macrophages, was obtained from thioglycolate-(2 ml 3% thioglycollate in sterile saline and filter-sterilized) induced intraperitoneal macrophages from 3 rats.
The total RNA was extracted using TRlzol reagent (Invitrogen) followed by further purification using RNeasy Mini Spin Columns (Qiagen). Total RNA was controlled for its integrity and purity using Agilent 2100 Bioanalyzer (Agilent Technologies) and Nanodrop spectrophotometer at the MicroArray Facility of the VIB (Flemish Interuniversitary Institute for Biotechnology), respectively. Probes were prepared from 5 µg total RNA, showing no signs of degradation or impurities (260/280 and 260/230 >1.8), according to Affymetrix's guidelines. Briefly, from total RNA, poly-A RNA was reversed transcribed using a poly dT-T7 primer and labeled during a T7 in-vitro transcription reaction using the Affymetrix IVT Labeling Kit (cat#900449, Affymetrix, High Wycombe, UK). The probes were purified (GeneChip Sample Cleanup Module, cat# P/N 900371, Affymetrix, UK) and analyzed again for yield (30-120µg) and purity (260/280 and 260/230 >1.8). 20µg was fragmented with alkaline hydrolysis. The fragmented aRNA was resuspended with control spikes in 300µl hybridization buffer (Eukaryotic Hybridization Control Kit, cat# 900299, Affymetrix, High Wycombe, UK) and 200 µl probe was hybridized in a rotisseri oven at 45 C. The genechips (Affymetrix GeneChip Rat Genome 230 2.0 Array, Affymetrix, UK) were washed and stained in the GeneChip Fluidics Station 400 (Affymetrix, UK) using EukGE-WS2v4 protocol, and subsequently scanned with the GeneChip Scanner 3000 (Affymetrix, UK). Image analysis was performed in GCOS.
The experiment was performed in triplicate using non-pooled samples obtained from a different animal. Image analysis was performed in GCOS. Probe intensity values reaching above background level with significance p < 0.05 were considered present calls. Functional analysis of the microarray data was performed using Onto-Express which classifies genes according to Gene Ontology categories. [Draghici et al (2003) Nucleic Acids Res. 31, 3775-3381]
As illustrated in example 3, a primary reaction occurs followed by a build up of both extracellular matrix molecules and homing proteins, providing cell attachment sites, at day three.
All the genes (±31000) present on the microarray chip were considered for the analysis. The venn-diagram (Figure 11) gives an overview of the gene expression finding. Comparing the expression profiles for FBR and IP, 3868 FBR3 and 2957 FBR1.5 specific genes (non-macrophage origin) were found. Genes of primary interest encode extracellular matrix proteins and signaling proteins enabling the attraction and homing of stem cells, which have been shown to be involved in immature FBR. Although a significant signal was found for structural molecules amongst which different collagens and laminins in both FBR1.5 and FBR3 the general expression of these molecules as grouped by GO only proved significant in the latter group. This means that regardless the expression of some structural molecules in the FBR1.5 group the significant contribution of those genes was only found in the FBR3 group, that is post cell homing. In FBR3 and FBR1.5 85 and 116 genes respectively were attributed to signal transducer activity GO term among which stromal cell derived factor 1 gamma (SDF-1), a molecule binding to haematopoietic stem cells and therefore an interesting candidate for integration in a biological matrix.
From these results appears that both SDF-1 and SCF were present in this adult tissue neogenesis and that both are candidate homing proteins to be studied for in vivo stem/progenitor cell homing to vascular/valvular prostheses.

### Example 4: Carotid artery grafts in rats.

The reseeding potential of the nanocoated materials was assessed by grafting a small calibre vascular graft into the common carotid artery as an interposition. The grafts were hand made out of photo oxidised pericardium with either SDF-1 or SCF at 1µg/per tube (in 30 µl PBS) with or without prior coating of the bovine pericardium with fibronectin. The graft remained in place for only 24h, sufficient to achieve cell adhesion but not enough to result in differentiation of the cells, which would result on loss of their stem cell properties. Tubes of bovine pericardium were prepared with the internal diameter approximating the internal diameter of a rat carotid artery. The graft was manufactured by rolling a small patch of photo oxidised over a small gauge plastic cannula and suturing the longitudinal edges using microsurgical techniques. The length of the graft was approximately 5mm and the internal diameter is 10 times smaller. Comparing the internal diameter of the graft to the internal diameter of the common carotid artery revealed that the graft's diameter is approximately 20% larger. This larger diameter was chosen because preliminary implants remained patent for several weeks.
The implantation protocol is an adaptation of the protocol for rabbits published by Boeckx (1997) Ann. Thorac. Surg. 63, S128-S134). Male Wistar rat (n = 6 for each material group) of 380 to 400 g were anaesthetised with isoflurane (induction: 4%; Surgery: 2%). After shaving and disinfecting with jodium alcohol, the common carotid artery was dissected free from the surrounding tissue and mounted in an Acland-type microclamp. The artery was then transected and both the proximal and distal of the graft construct were sutured with a 10/0 monofilament nylon, using the 7 o'clock stitch technique (Kirsch et al. (1992) Am. Surg. 58, 722-727) which requires 9 to 12 stitches. The needle catched the full thickness of the vessel wall. Care was taken that only the needle touches the intima of the artery. The whole procedure was performed without spasmolytica (e.g. papaverin) and anticoagulantia (e.g. heparin). After the last stitch the double Acland-type microclamp was removed. After a few minutes to assure complete haemostasis the skin was closed. The anaesthesia was discontinued and approximately 5 minutes later the animal awakened and was transferred to an individual cage.

After 24 h the wound was reopened and the graft was prelevated and washed with phosphate buffered saline. The graft and on each anastomosis a small portion of the native carotid artery was excised. The lumen was gently flushed with phosphate buffered saline and subsequently filled with Tissue Freezing medium. After being embedded in the same medium the sample was snap-frozen in liquid nitrogen and stored at -80°C. Sectioning is performed on a Microm HM500 OM cryostat (Prosan, Merelbeke, Belgium). The 7 µm longitudinal sections were placed on poly-L-lysine coated slides and stored at - 20 until staining.
Emphasis was put on stem cell attraction, therefore the samples were immunohistochemically stained for 4 markers: CD3 (BD Pharmingen; clone G4.18), c-kit (Santa Cruz Biotechnology; clone H-300), CD34 (DAKO; clone QBEnd 10), Sca-1 (R&D Systems; goat polyclonal). The Table below indicates the cell types stained for by each antibody.

**Table 4: Antibodies used for cell staining**

| Marker | Synonyms | Celtypes |
|---|---|---|
| CD3 | | T-cell (Nicolls et al. cited above) |
| CD117 | c-kit, SCF-receptor | Stem cell subset (Buhring et al. cited above; Gehling et al. cited above) |
| CD34 | | Haematopoietic progenitor cells ( Askari et al. (2003) Lancet 362, 697-703.; Okada et al. cited above), circulating immature cells (Taguchi et al. cited above), mesenchymal stem cells (Rombouts and Ploemacher cited above) |
| Sca-1 | Stem cell antigen | Stem cells (Krause et al. cited above) |

All primary antibodies were detected with a fluorochrome, FITC or texas red, conjugated antibody. The image analysis was performed using an Axioplan 2 imaging microscope (Zeiss, Zaventem, Belgium) and the Axiovision 4.4 software package (Zeiss, Zaventem, Belgium). For cell phenotyping a total of 250 cells were assessed for each longitudinal cross section and the results were expressed as a percentage. To avoid bias several pictures were taken, quartered and counted according to a randomisation list.
Overall no difference in quantitative cell adhesion was found (Table 5)

**Table 5: Cell count data**

| | Control | SCF | FN+SCF | SDF-1 | FN+SDF-1 |
|---|---|---|---|---|---|
| Cell count | 2276 | 3212 | 1299 | 2316 | 1697 |
| | [158, 5490] | [1293, 4324] | [1044, 2812] | [1619, 3359] | [969, 2861] |

CD34 positive cells were found in two groups. These cells were present in the controls (1.85 [0.00, 7.21]%) which were only subjected to spontaneous seeding after implantation in the rat's blood vessel. Furthermore they showed to be present in SCF impregnated photoxidised bovine pericardium (3.84 [0.00, 11.08]%), althought a numerical increase was found this was not significant due to the interindividual large variation. The three remaining groups did not contain CD34+ cells.

The results of the CD117 immunostaining are shown in Figure 12(A). The control samples comprised of photooxidised pericardium showed an median presence of 4.70 [2.14, 12.17]% CD117⁺ cells after being implanted in the carotid artery of a rat. Impregnating the same matrix material with either SCF or SDF-1 significantly increased the fraction of CD117⁺ cells in and on the luminal side of the implants. A 15.78 [10.04, 47.90]% and 34.02 [26.32, 37.39]% fraction was found for SCF and SDF-1 respectively. Although fibronectine co-impregnation did not have an effect on the presence of CD34⁺ and Sca-1⁺ cells a clear increase in the homing of CD117⁺ cells was found. Co-impregnation of fibronectine and SCF resulted in a 47.84 [41.10,66.00]% CD117⁺ cell fraction. Although a large numerical increase this was found to be not significant due to the large variation in the CD117⁺ fraction in the SCF group. On the other hand the increase found in the CD117⁺ fraction in the fibronectin and SDF-1 coimpregnated group (48.90 [42.32, 54.08]%) was found to be significantly increased when compared to the SDF-1 group. In general it was found that all of the impregnation protocols induced enhanced homing of CD117 positive cells and that especially the combinations of either SCF or SDF-1 with fibronectine resulted in about 50% of the cells to be positive for this marker.

Finally, the attraction of Sca-1+ stem cells to photooxidised bovine pericardium coated with SDF-1 or SCF with or without precoating with fibronectin was investigated (see Figure 12b). In the control some Sca-1 positive cells were found in 2 out of 6 implants. When the same matrix material was impregnated this resulted in Sca-1 positive cell adhesion in al implants as well as a significant increase in the percentage of Sca-1 positive cells. Sca-1 positive cells can be attributed to either the stem cell group or to a subset of T-cells. The staining for T-cells (anti CD3 immunohistochemistry) was shown to be negative in all implants thereby confirming the specific homing of Sca-1 positive stem cells to the impregnated material.

### Example 5: Patches implanted in the carotid artery of sheep.

SCF and SDF-1 impregnated bovine pericardial patches have been implanted in the sheep carotid artery. Both proteins have been used with or without prior coating of the matrix material with fibronectin. Four patches have been implanted in each (left and right) carotid artery. In each side a control, 1µg, 3µg and 10µg per cm² coated patch were implanted. The control was implanted downstream and subsequently the 1, 3 and 10µg/cm² patches were implanted with the 10µg/cm² patch in the most upstream position. Figure 13 shows that some CD34+ cells (haematopoietic stem/progenitor cells) were found.

## Claims

1. Use of a homing factorcapable of binding to stem cells or progenitor cells in the preparation of a scaffold having a structural matrix, wherein the use of said scaffold with a homing factor is for direct *in situ in vivo* cell seeding, and wherein said homing factor is a ligand of a receptor, or a fragment of said ligand comprising the receptor binding domain, or a peptide which binds to the receptor.

2. A method for preparing for direct *in situ in vivo* cell seeding, a scaffold having a structural matrix, said method comprising the step of applying to said structural matrix a homing factor capable of binding to stem cells or progenitor cells, wherein said homing factor is a ligand of a receptor, or a fragment of said ligand comprising the receptor binding domain, or a peptide which binds to the receptor

3. The use according to claim 1 or the method according to claim 2, wherein said ligand is selected from the group consisting of stromal derived factor 1, stem cell factor, VCAM-1, P1 region of fibrinogen and P2 region of fibrinogen.

4. The use according to claim 1 or the method according to claim 2, wherein said ligand is stromal derived factor 1,

5. The use or method according to any one of claims 1 to 4, for direct in vivo recellularisation under conditions of high shear stress.

6. The use or method according to any of claims 1 to 5, wherein said scaffold is a scaffold of a blood vessel or cardiac valve.

7. The use or method according to any one of claims 1 to 6, wherein the matrix is precoated with one or more proteins facilitating the interaction between the ligand or fragment thereof and the structural matrix.

8. The use or method according to claim 7, wherein the protein facilitating the matrix is selected from the group consisting of fibronectin, collagen and fibrinogen.

9. The use or method according to claim 7, wherein the protein facilitating the matrix is fibronectin.

10. The use or method according to any one of claims 1 to 9, wherein the structural matrix is a non-crosslinked prosthesis or acellularised aortic root.

11. The use or method according to any one of claims 1 to 10, wherein the homing factor is applied on the structural matrix by chemical cross-linking or by impregnating the matrix with a solution comprising the homing factor.

## Patentansprüche

1. Verwendung eines Homing-Faktors, der imstande ist, an Stammzellen oder Vorläuferzellen zu binden, in der Herstellung eines Gerüsts mit einer Strukturmatrix, wobei die Verwendung des Gerüsts mit einem Homing-Faktor der direkten In-situ-Zellaussaat in vivo dient und wobei der Homing-Faktor ein Ligand eines Rezeptors ist oder ein Fragment des Liganden, das die Rezeptorbindungsdomäne umfasst, oder ein Peptid, das an den Rezeptor bindet.

2. Verfahren zur Herstellung eines Gerüsts mit einer Strukturmatrix zur direkten In-situ-Zellaussaat in vivo, wobei das Verfahren den Schritt des Anwendens eines Homing-Faktors der imstande ist, an Stammzellen oder Vorläuferzellen zu binden, bei der Strukturmatrix umfasst, wobei der Homing-Faktor ein Ligand eines Rezeptors ist oder ein Fragment des Liganden, das die Rezeptorbindungsdomäne umfasst, oder ein Peptid, das an den Rezeptor bindet.

3. Verwendung nach Anspruch 1 oder Verfahren nach Anspruch 2, wobei der Ligand ausgewählt ist aus der Gruppe bestehend aus Stromal-derived Factor 1, Stammzellenfaktor, VCAM-1, P1-Region von Fibrinogen und P2-Region von Fibrinogen.

4. Verwendung nach Anspruch 1 oder Verfahren nach Anspruch 2, wobei der Ligand Stromal-derived Factor 1 ist.

5. Verwendung oder Verfahren nach einem der Ansprüche 1 bis 4, zur direkten In-Vivo-Rezellularisierung unter Bedingungen hoher Scherbelastungen.

6. Verwendung oder Verfahren nach einem der Ansprüche 1 bis 5, wobei das Gerüst ein Gerüst aus einem Blutgefäß oder einer Herzklappe ist.

7. Verwendung oder Verfahren nach einem der Ansprüche 1 bis 6, wobei die Matrix mit einem oder mehreren Proteinen vorbeschichtet ist, wodurch die Wechselwirkung zwischen dem Liganden oder Fragment davon und der Strukturmatrix erleichtert wird.

8. Verwendung oder Verfahren nach Anspruch 7, wobei das Protein, das die Matrix erleichtert, ausgewählt ist aus der Gruppe bestehend aus Fibronectin, Collagen und Fibrinogen.

9. Verwendung oder Verfahren nach Anspruch 7, wobei das Protein, das die Matrix erleichtert, Fibronectin ist.

10. Verwendung oder Verfahren nach einem der Ansprüche 1 bis 9, wobei die Strukturmatrix eine nicht vernetzte Prothese oder azellularisierte Aortenwurzel ist.

11. Verwendung oder Verfahren nach einem der Ansprüche 1 bis 10, wobei der Homing-Faktor bei der Strukturmatrix durch chemisches Vernetzen oder durch Imprägnieren der Matrix mit einer Lösung, die den Homing-Faktor umfasst, angewendet wird.

## Revendications

1. Utilisation d'un facteur d'écotropisme susceptible de se lier à des cellules souches ou des cellules progénitrices dans la préparation d'un échafaudage ayant une matrice structurelle, dans laquelle l'utilisation dudit échafaudage avec un facteur d'écotropisme sert à l'ensemencement direct *in situ* de cellules *in vivo,* et dans laquelle ledit facteur d'écotropisme est un ligand d'un récepteur, ou un fragment dudit ligand comprenant le domaine de liaison au récepteur, ou un peptide qui se lie au récepteur.

2. Procédé pour la préparation, pour un ensemencement direct *in situ* de cellules *in vivo,* d'un échafaudage ayant une matrice structurelle, ledit procédé comprenant l'étape d'application à ladite matrice structurelle d'un facteur d'écotropisme susceptible de se lier à des cellules souches ou des cellules progénitrices, dans lequel ledit facteur d'écotropisme est un ligand d'un récepteur, ou un fragment dudit ligand comprenant le domaine de liaison au récepteur, ou un peptide qui se lie au récepteur

3. Utilisation selon la revendication 1 ou procédé selon la revendication 2, dans lesquels ledit ligand est sélectionné à partir du groupe constitué par lefacteur 1 tiré de cellules stromales, d'un facteur de cellule souche, VCAM-1, la région P1 du fibrinogène et la région P2 du fibrinogène.

4. Utilisation selon la revendication 1 ou procédé selon la revendication 2, dans lesquels ledit ligand est lefacteur 1 dérivé de cellules stromales.

5. Utilisation ou procédé selon l'une quelconque des revendications 1 à 4, pour la recellularisation directe in vivo sous des conditions de contrainte de cisaillement élevée.

6. Utilisation ou procédé selon l'une quelconque des revendications 1 à 5, dans lesquels ledit échafaudage est un échafaudage d'un vaisseau sanguin ou d'une valvule cardiaque.

7. Utilisation ou procédé selon l'une quelconque des revendications 1 à 6, dans lesquels la matrice est revêtue à l'avance par une ou plusieurs protéines facilitant l'interaction entre le ligand ou fragment de ce dernier et la matrice structurelle.

8. Utilisation ou procédé selon la revendication 7, dans lesquels la protéine facilitant la matrice est sélectionnée à partir du groupe constitué par la fibronectine, le collagène et le fibrinogène.

9. Utilisation ou procédé selon la revendication 7, dans lesquels la protéine facilitant la matrice est la fibronectine.

10. Utilisation ou procédé selon l'une quelconque des revendications 1 à 9, dans lesquels la matrice structurelle est une prothèse non réticulée ou une racine aortique acellularisée.

11. Utilisation ou procédé selon l'une quelconque des revendications 1 à 10, dans lesquels le facteur d'écotropisme est appliqué sur la matrice structurelle par réticulation chimique ou en imprégnant la matrice avec une solution comprenant le facteur d'écotropisme.
